Europäisches Patentamt

European Patent Office

Office européen des brevets

(11) Publication number: **0 165 740**
**B1**

# EUROPEAN PATENT SPECIFICATION

(45) Date of publication of patent specification: **30.08.89**

(51) Int. Cl.⁴: **A 61 M 5/14**

(21) Application number: **85303992.3**

(22) Date of filing: **05.06.85**

(54) Fluid flow sensor.

(30) Priority: **18.06.84 US 621573**

(43) Date of publication of application:
**27.12.85 Bulletin 85/52**

(45) Publication of the grant of the patent:
**30.08.89 Bulletin 89/35**

(84) Designated Contracting States:
**DE FR GB**

(56) References cited:
**WO-A-82/01997**
**FR-A-2 316 509**
**FR-A-2 360 290**
**NL-A-7 106 111**

(73) Proprietor: **PARKER HANNIFIN CORPORATION**
**17325 Euclid Avenue**
**Cleveland Ohio 44112 (US)**

(72) Inventor: **Pool, Scott L.**
**510 W. Bell**
**Santa Ana California 92707 (US)**

(74) Representative: **Purvis, William Michael**
**Cameron et al**
**D. Young & Co. 10 Staple Inn**
**London WC1V 7RD (GB)**

Courier Press, Leamington Spa, England.

## Description

The invention relates to fluid flow sensors and particularly, though not exclusively, to flow sensors suitable for use in medication dispensing systems.

Over the years, various types of medication dispensing systems have been developed. Generally, these systems provide for delivery of medication from a variety of pressure chambers or pumps through a catheter and into the patient. Examples are shown and described in U.S.A. Patents Nos. 3,951,147; 4,193,397; 4,360,019 and 4,373,527. Such systems are, however, subject to various failure modes such as a stalled pump condition, or an occlusion in the catheter. Such failures have been particularly troublesome where the delivery rate of medication is so low that user is unaware of the interruption of medication flow until physiological symptoms develop.

Accordingly, there was a need in the prior art for a device that would indicate the fluid flow condition in various types of medication delivery systems. Because of the potentially harmful consequences of interruptions in medication delivery, it was most important that such a flow sensor be reliable. However, such sensors would typically be applied to systems having at least some disposable components so that it was also important that the flow sensor could be inexpensively manufactured. Moreover, many such systems are carried by the patients on a daily basis in the normal course of their activities. These systems are compact and highly portable so that a compatible flow sensor would also need to be similarly compact and portable.

Various types of fluid pressure measuring devices are known from the prior art. For example, U.S.A. Patent No. 4,398,542 is directed to a pressure measuring device wherein pressure of fluid flow between a flexible membrane and a body is sensed by a pressure transducer. Other examples of pressure measuring devices are shown in U.S.A. Patents Nos. 4,185,641; 3,240,207; and 1,861,999. However, none of those devices affords a suitably reliable yet inexpensive flow sensor that is sufficiently compact for use in a wide range of medication dispensing systems.

Some medication delivery systems have employed various schemes for providing audible or visual alarms in response to over-pressure conditions. However, these were pressure-sensitive arrangements that were not responsive to flow conditions. Indeed, they generally required a substantial pressure accumulation to activate the alarm. Moreover, the benefit of such alarms was seriously compromised for patients having visual or auricular disabilities.

Consequently, various methodologies for detecting medication flow were developed. For example, the use of a portable medication dispensing device would occasionally remove the needle from his body to observe whether medication was, in fact, flowing. Such methodologies were, at best uncomfortable and awkward for the user. Moreover, they tended to expose the medication system to contamination and the patient to infection.

There was thus a need for a flow sensor that was inexpensive, reliable, and suitable for use on medication infusion devices, preferably with the ability to provide a tangibly perceptive indication of flow conditions.

According to the invention there is provided a fluid flow sensor comprising:

a body with an input end and an output end, a first internal passage and a second internal passage in the body and a flexible cover secured to the body, the flexible cover being movable between a contracted position and an expanded position in response to fluid flow through the first and second internal passages;

characterised in that the body has a recessed surface located between the input end and the output end, the first internal passage is located between the input end and the recessed surface and the second internal passage is located between the output end and the recessed surface; and that the flexible cover contacts the recessed surface in the contracted position and is spaced apart from the recessed surface in the expanded position.

Preferably, the flexible cover is tensioned such that it is normally urged toward the contracted position where it contacts the recessed surface, but is maintained in an expanded position where it is apart from the recessed surface in response to fluid flow through the internal passages. Under normal fluid flow conditions, the expanded position of the flexible cover is generally located at a radius intermediate the radius of the peripheral surface of the body and the radius of the recessed surface. Under occluded fluid flow conditions, the expanded position of the flexible cover is generally located at a radius adjacent the radius of the peripheral surface of the body.

Preferably, the body has a generally cylindrical shape with the recessed area being a saddle-shaped surface having a planar central surface with adjoining convex surfaces at oppositely disposed edges. Also preferably, the fluid flow sensor includes tubes that are respectively secured by adhesive in recesses in the input and output ends of the body and communicate with the first and second passages, the cover being secured to the tubes by adhesive.

The invention is diagrammatically illustrated by way of example with reference to the accompanying drawings, in which:—

Figure 1 is a perspective view of a fluid flow sensor according to the invention;

Figure 2 is a cross-section of the sensor of Figure 1 taken along longitudinal axis A—A' and showing the cover in a contracted state;

Figure 3 is a similar cross-section to that of Figure 2 but showing the cover in an expanded state that indicates normal flow; and

Figure 4 is a similar cross-section to that of Figure 2 except that the cover is shown in an expanded state that indicates occluded flow.

Referring to the drawing, a fluid flow sensor has a body 10 enveloped in a flexible cover 12. While the body 10 can have various other general shapes and sizes, in the preferred embodiment, the body 10 is of a generally cylindrical shape aligned along a central longitudinal axis A—A' and is formed of a polycarbonate material. The body 10 includes an input end 14 and an output end 16 at oppositely disposed, longitudinal ends thereof. The body 10 also includes a generally cylindrical peripheral surface that is located at a radius $R_2$ from the central axis A—A'.

The body 10 further includes a recessed surface 18 having a planar surface 20 and adjoining convex surfaces 22 and 24. The planar surface 20 is tangentially located with respect to the central longitudinal axis A—A' at a radius $R_1$ that is less than the radius $R_2$. The planar surface 20 intersects the cylindrical surface of the body 10 and adjoins the convex surfaces 22 and 24 at oppositely disposed sides thereof. The convex surfaces 22 and 24 provide a continuous surface between the adjoining edge of the planar surface 20 and the peripheral surface of the body 10 such that the recessed surface 18 forms a saddle-shaped surface.

The body 10 further includes recesses 26 and 28 respectively located in the input end 14 and the output end 16. Passages 30 and 32 are also included in the body 10. The passage 30 provides fluid communication between the recessed surface 18 and the recess 26 and the passage 32 provides fluid communication between the recessed surface 18 and the recess 28. More specifically, the passage 30 intersects the recessed surface 18 to form a port 34 in the planar surface 20 adjacent the intersection with the convex surface 22 and the passage 32 intersects the recessed surface 18 to form a port 36 in the planar surface 20 adjacent the intersection with the convex surface 24.

Tubes 38 and 40 are secured in the recesses 26 and 28 respectively to link the disclosed flow sensor to the rest of the fluid system. Thus, the passage 30 provides fluid communication between the recessed surface 18 and the tubes 38 and the passage 32 provides fluid communication between the recessed surface 18 and the tube 40.

The flexible cover 12 is formed of a medical-grade, heat-shrink tubing selected from a group of materials including polyvinyl chloride. The flexible cover 12 envelops the body 10 and is secured thereto through a commercially known heat-shrinking process such that its constricts about the body 10 and is under tension across the recessed surface 18. The flexible cover 12 is a thin-wall material having a specified tensile strength and elasticity over a range of intended flow conditions for the fluid stream.

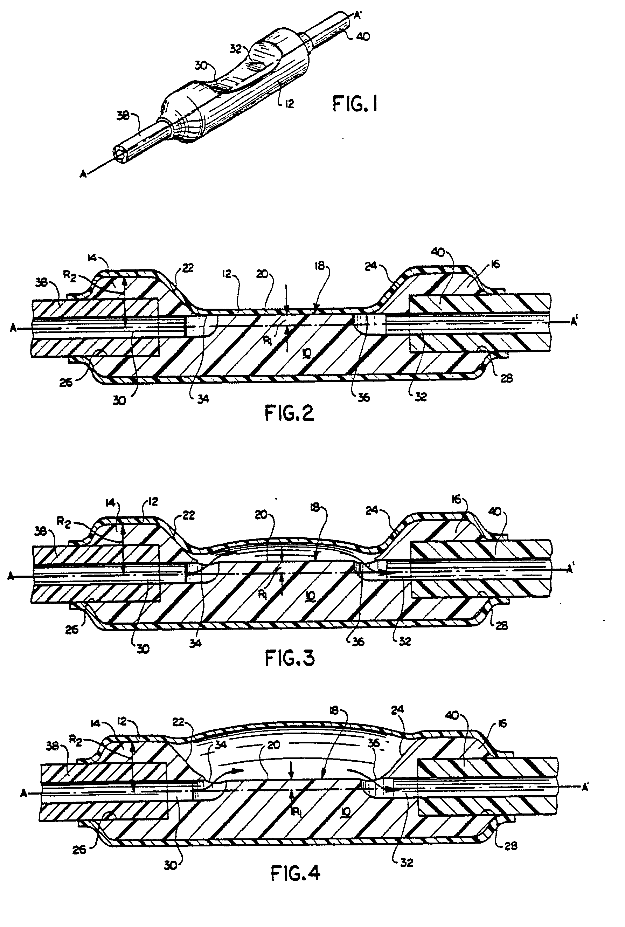

As particularly shown in Figures 2, 3 and 4, the flexible cover 12 is movable between a contracted position and an expanded position. Figure 2 shows the contracted position of the flexible cover 12 wherein it contacts the recessed surface 18 between the ports 34 and 36 and is substantially parallel to the planar surface 20. Thus, with the flexible cover 12 in the contracted position, the flexible cover 12 is substantially planar and there is no path for fluid flow between the port 34 of the passage 30 and the port 36 of the passage 32.

Under normal flow conditions, the fluid separates the flexible cover 12 from contact with the recessed surface 18 as shown in Figure 2, and moves it into an expanded position in which it is spaced from the recessed surface 18 as shown in Figure 3. In the expanded position of Figure 3, the flexible cover 12 is spaced from the planar surface 20 and has a generally convex of dome shape. In this expanded position, the flexible cover 12 is located at a radius intermediate the radius $R_2$ of the peripheral surface of the body 10 and the radius $R_1$ of the recessed surface 18. Thus, a flow path between the ports 34 and 36 is established.

Under occluded flow conditions, the fluid separates the flexible cover 12 from contact with the recessed surface 18 and moves it into an expanded position spaced from the recessed surface 18 as shown in Figure 4. In Figure 4, the flexible cover 12 is generally at a radius adjacent the radius $R_2$ of the peripheral surface of the body 10.

Accordingly, flow conditions through the disclosed sensor can be detected by monitoring the position of the flexible cover 12 in the region of the recessed surface 18. When the flexible cover 12 is continuously adjacent the recessed surface 18, there is no flow, but when the flexible cover 12 is expanded away from recessed surface 18, the sensor detects normal or occluded flow conditions.

In the operation of the preferred embodiment, fluid flow in the fluid path in which the sensor is incorporated causes substantial movement of the flexible cover 12 such that flow conditions can be monitored manually. Specifically, a patients holds the sensor between his thumb and index finger to sense the relative movement of the flexible cover 12 with respect to the body 10. A convex shape of the cover 12 in the region of the planar surface 20 of recessed surface 18 indicates that flow conditions exist in the sensor. A planar shape of the cover 12 in the region of planar surface 20 indicates that fluid flow conditions do not exist.

Thus, as applied to intermittent or pulsed-flow systems, the disclosed sensor will indicate that no flow is established at the pump by remaining constantly planar as shown in Figure 2. However, the sensor will indicate that normal or expected flow conditions are established through the fluid path being monitored by pulsing between its contracted position shown in Figure 2 and its expanded position shown in Figure 3 in accordance with the pulsed flow. The disclosed sensor will indicate a downstream occlusion of the flow path by constantly remaining in its expanded position as shown in Figure 4.

Claims

1. A fluid flow sensor comprising:

a body (10) with an input end (14) and an output end (16), a first internal passage (30) and a second internal passage (32) in the body (10) and a flexible cover (12) secured to the body (10), the flexible cover (12) being movable between a contracted position and an expanded position in response to fluid flow through the first (30) and second (32) internal passages;

characterised in that the body (10) has a recessed surface (18) located between the input end (14) and the output end (16), the first internal passage (30) is located between the input end (14) and the recessed surface (18) and the second internal passage (32) is located between the output end (16) and the recessed surface (18); and that the flexible cover (12) contacts the recessed surface (18) in the contracted position and is spaced apart from the recessed surface in the expanded position.

2. A fluid flow sensor according to claim 1, wherein the first internal passage (30) forms a first port (34) in the recessed surface (18) and the second internal passage (32) forms a second port (36) in the recessed surface (18), and wherein the flexible cover (12) is tensioned such that it is normally urged toward contact with the recessed surface (18) between the first (34) and second (36) ports.

3. A fluid flow sensor according to claim 1 or claim 2, wherein the flexible cover (12) is secured to the body (10) adjacent the perimeter of the recessed surface (18).

4. A fluid flow sensor according to claim 2, wherein the recessed surface (18) is a saddle-shaped surface comprising:

a planar surface (20); and

first (22) and second (24) convex surfaces that adjoin the planar surface (20) at oppositely disposed edges thereof.

5. A fluid flow sensor according to claim 4, wherein the first (34) and second (36) ports are respectively located adjacent the junctions between the planar surface (20) and the first (22) and second (24) convex surfaces.

6. A fluid flow sensor according to any one of claims 1 to 5, further comprising:

first (38) and second (40) tubes that are respectively secured to the input (14) and output (16) ends of the body (10), and are respectively in communication with the first (30) and second (32) passages.

7. A fluid flow sensor according to claim 6, wherein the first (38) and second (40) tubes are secured to the body (10) by adhesive.

8. A fluid flow sensor according to claim 6, wherein the flexible cover (12) is secured to the first (38) and second (40) tubes.

9. A fluid flow sensor according to any one of claims 1 to 8, wherein the expanded position of the flexible cover (12) is located at a radius adjacent the radius ($R_2$) of the peripheral surface of the body (10) during occluded flow conditions.

10. a fluid flow sensor according to any one of claims 1 to 9, wherein the expanded position of the flexible cover (12) under normal flow conditions is located at a radius intermediate the radius ($R_2$) of the peripheral surface of the body and the radius ($R_1$) of the recessed surface (18).

11. A fluid flow sensor according to claim 18, wherein the flexible cover (12) is generally dome-shaped in its expanded position.

12. A fluid flow sensor according to any one of claims 1 to 11, wherein the body (10) is formed of a polycarbonate material and the cover (12) is of thin wall polyvinyl chloride tubing.

Patentansprüche

1. Ein Durchflußsensor, der aufweist:

einen Körper (10) mit einem Zuflußende (14) und einem Ausflußende (16), einem ersten inneren Durchlaß (30) und einem zweiten inneren Durchlaß (32) im Körper (10) und einem flexiblen, mit dem Körper (10) fest verbundenen Deckel (12), wobei der flexible Deckel (12) in Reaktion auf Fluidströme durch den ersten inneren Durchlaß (30) und den zweiten (32) inneren Durchlaß zwischen einer zurückgezogenen Lage und einer ausgefahrenen Lage beweglich ist, dadurch gekennzeichnet, daß der Körper (10) eine abgesenkte Fläche (18) aufweist, die zwischen dem Zuflußende (14) und dem Ausflußende (16) liegt, dadurch, daß der erste innere Durchlaß (13) zwischen dem Zuflußende (14) und der abgesenkten Fläche (18) liegt und der zweite innere Durchlaß (32) zwischen dem Ausflußende (16) und der abgesenkten Fläche (18) liegt, und dadurch, daß der flexible Deckel (12) in der zurückgezogenen Lage die abgesenkte Fläche (18) berührt und in der ausgefahrenen Lage von der abgesenkten Fläche (18) räumlich getrennt ist.

2. Durchflußsensor nach Anspruch 1, bei dem der erste innere Durchlaß (30) eine erste Öffnung (34) in der abgesenkten Fläche (18) bildet und der zwzite innere Durchlaß (32) eine zweite Öffnung (36) in der abgesenkten Fläche (18) bildet, und wobei der flexible Deckel (12) in der Weise unter Spannung gesetzt wird, daß er normalerweise in Richtung auf eine Berührung mit der angesenkten Fläche (18) zwischen der ersten Öffnung (34) und der zweiten Öffnung (36) gedrückt wird.

3. Durchflußsensor nach Anspruch 1 oder 2, bei dem der flexible Deckel (12) mit dem Körper (10) neben der Umfangslinie der abgesenkten Fläche (18) befestigt ist.

4. Durchflußsensor nach Anspruch 2, bei dem die abgesenkte Fläche (18) eine sattelförmige Fläche ist mit: einer planen Fläche (20) und einer ersten konvexen Fläche (22) und einer zweiten konvexen Fläche (24), die neben der planen Fläche (20) an gegenüberliegend angeordneten Kanten derselben anliegt.

5. Durchflußsensor nach Anspruch 4, bei dem die erste Öffnung (34) und die zweiten Öffnung (36) jeweils neben den Verbindungen zwischen der planen Fläche (20) und der ersten konvexen Fläche (22) und der zweiten konvexen Fläche (24) angeordnet sind.

6. Durchflußsensor nach einem der Ansprüche 1 bis 5, der des weiteren aufweist: erste (38) und zweite (40) Rohre, die jeweils an dem Zuflußende (14) und dem Ausflußende (16) des Körpers (10) angeordnet sind und jeweils mit dem ersten Durchlaß (30) und dem zweiten Durchlaß (32) verbunden sind.

7. Durchflußsensor nach Anspruch 6, bei dem das erste (38) und zweite (40) Rohr durch Klebstoff am Körper (10) befestigt sind.

8. Durchflußsensor nach Anspruch 6, bei dem der flexible Deckel (12) an dem ersten (38) und zweiten (40) Rohr angeschlossen ist.

9. Durchflußsensor nach einem der Ansprüche 1 bis 8, bei dem die ausgefahrene Lage des flexiblen Deckels (12) während des Zustandes der Durchflußsperre bei einem Radius liegt, der neben dem Radius ($R_2$) der peripherischen Fläche des Körpers (10) angeordnet ist.

10. Durchflußsensor nach einem der Ansprüche 1 bis 9, bei dem die ausgefahrene Stellung des flexiblen Deckels (12) unter normalen Durchflußbedingungen an einem Radius liegt, der zwischen dem Radius ($R_2$) der peripherischen Fläche des Körpers und dem Radius ($R_1$) der angesenkten Fläche (18) verläuft.

11. Durchflußsensor nach Anspruch 10, bei dem der flexible Deckel in seiner ausgefahrenen Lage im allgemeinen domförmig ist.

12. Durchflußsensor nach einem der Ansprüche 1 bis 11, bei dem der Körper (10) aus einem Polykarbonat-Material und der Deckel (12) aus dünnwandigen Polyvinylchlorid-Rohren besteht.

**Revendications**

1. Capteur de débit comprenant: un corps (10) muni d'une extrémité d'entrée (14) et d'une extrémité de sortie (16), un premier passage intérieur (30) et un second passage intérieure (32) dans le corps (10) et un capot flexible (12) fixé au corps (10), le capot flexible (12) étant mobile entre une position contractée et une position dilatée en réponse au débit de fluide passant dans le premier (30) et second 32) passage intérieur; caractérisé en ce que le corps (10) comporte une surface renfoncée (18) située entre l'extrémité d'entrée (14) et l'extrémité de sortie (16), le premier passage intérieur (30) étant situé entre l'extrémité d'entrée (14) et la surface renfoncée (18) et le second passage intérieur (32) étant situé entre l'extrémité de sortie (16) et la surface renfoncée (18), et en ce que le capot flexible (12) vient en contact avec la surface renfoncée (18) dans la position contractée et se trouve écarté de la surface renfoncée dans la position dilatée.

2. Capteur de débit selon la revendication 1, caractérisé en ce que le premier passage intérieur (30) forme un premier orifice (34) dans la surface renfoncée (18), et le second passage intérieur (32) forme un second orifice (36) dans la surface renfoncée (18), et en ce que le capot flexible (12) et tendu de manière à être normalement poussé en contact avec la surface renfoncée (18) entre le premier orifice (34) et le second orifice (36).

3. Capteur de débit selon l'une quelconque des revendications 1 et 2, caractérisé en ce que le capot flexible (12) est fixé au corps (10) au voisinage du périmètre de la surface renfoncée (18).

4. Capteur de débit selon la revendication 2, caractérisé en ce que la surface renfoncée (18) est une surface en forme de selle comprenant une surface plane (20); et une première surface convexe (22) et une seconde surface convexe (24) venant se relier à la surface plane (20) sur les bords opposées de celle-ci.

5. Capteur de débit selon la revendication 4, caractérisé en ce que le premier orifice (34) et le second orifice (36) sont placés respectivement au voisinage des jonctions entre la surface plane (20) et la première (22) et seconde (24) surface convexe.

6. Capteur de débit selon l'une quelconque des revendications 1 à 5, caractérisé en ce qu'il comprend en outre un premier tube (38) et un second tube (40) fixés respectivement à l'extrémité d'entrée (14) et à l'extrémité de sortie (16) du corps (10), et se trouvant respectivement en communication avec le premier passage (30) et le second passage (32).

7. Capteur de débit selon la revendication 6, caractérisé en ce que le premier tube (38) et le second tube (40) sont fixés au corps (10) par collage.

8. Capteur de débit selon la revendication 6, caractérisé en ce que le capot flexible (12) est fixé au premier tube (38) et au second tube (40).

9. Capteur de débit selon l'une quelconque des revendications 1 à 8, caractérisé en ce que la position dilatée du capot flexible (12) est située à un rayon voisin du rayon ($R_2$) de la surface périphérique du corps (10) pendant les conditions de fermeture du débit.

10. Capteur de débit selon l'une quelconque des revendications 1 à 9, caractérisé en ce que la position dilatée du capot flexible (12), dans les conditions de débit normales, est située à un rayon intermédiare, entre le rayon ($R_2$) de la surface périphérique du corps et le rayon ($R_1$) de la surface renfoncée (18).

11. Capteur de débit selon la revendication 10, caractérisé en ce que le capot flexible (12) est en forme générale de dôme dans sa position dilatée.

12. Capteur de débit selon l'une quelconque des revendications 1 à 11, caractérisé en ce que le corps (10) est réalisée dans un matériau de polycarbonate et en ce que le capot (12) est constitué par un tube mince en chlorure de polyvinyle.

FIG. 1

FIG. 2

FIG. 3

FIG. 4